# EUROPÄISCHE PATENTANMELDUNG

(11) **EP 1 020 758 A1**
(43) Veröffentlichungstag der Anmeldung: **19.07.2000**
(21) Anmeldenummer: 99100343.5
(22) Anmeldetag: 14.01.1999
(51) Int. Cl.: G03B 42/04

(54) **Intraorale Halterung für einen Sensor und Verfahren zum Verbinden eines Sensors mit einer intraoralen Halterung**

(71) Anmelder: Schäfer, Klaus, Dr. med. dent., 59067 Hamm (DE)
(72) Erfinder: Schäfer, Klaus, Dr. med. dent., 59067 Hamm (DE)
(74) Vertreter: Thul, Hermann, Dipl.-Phys.

(57) **Zusammenfassung**

Die Erfindung betrifft eine intraorale Halterung (10) mit einer Bißplatte (130), wobei die Halterung (10) für eine Lagerung eines Sensors (40) dient.

Erfindungsgemäß zeichnet sich die Halterung (10) dadurch aus, daß sie wenigstens zwei im wesentlichen senkrecht zu der Bißplatte (130) angeordnete Vorsprünge (20, 30) aufweist.

## Beschreibung

Die Erfindung betrifft eine intraorale Halterung mit einer Bißplatte, wobei die Halterung zu einer Lagerung eines Sensors dient.

Durch offenkundige Vorbenutzung sind Haltesysteme für Röntgensysteme bekannt, durch die sich mit Röntgenfilmen verschiedene Röntgenaufnahmetechniken realisieren lassen. Bei den Röntgenaufnahmetechniken handelt es sich insbesondere um die sogenannte Rechtwinkel-/Paralleltechnik, eine Kombination aus einer Rechtwinkeltechnik und einer Paralleltechnik. Die Rechtwinkel-/Paralleltechnik ermöglicht Röntgenaufnahmen mit einem Zahnfilm innerhalb der Mundhöhle.

Bei der Paralleltechnik werden Zahnachse und Filmachse parallel zueinander ausgerichtet. Trifft ein Zentralstrahl einer Röntgenguelle, insbesondere einer Röntgenröhre, auf die Mitte des Zahnes, trifft er ebenso auf die Mitte des Filmes. Die parallele Ausrichtung von Zahnachse und Filmachse gewährleistet eine Ablichtung des Zahnes auf den Film in einer im wesentlichen unveränderten Größe. Diese Aufnahmetechnik ist jedoch mit dem Nachteil einer ungenauen Justierung des Röntgentubus auf die Filmmitte sowie einer ungenauen Winkelstellung des Tubus zu dem Zahn und dem Film behaftet.

Bei der Rechtwinkeltechnik wird die Halterung so gestaltet, daß sie mit einem Tubus starr verbunden ist, so daß der Film im rechten Winkel zu dem Zentralstrahl des Röntgengerätes fixiert ist. Hierdurch trifft der Zentralstrahl stets im Winkel von 90° die Filmmitte. Diese Röntgenaufnahmetechnik hat den Vorteil, daß eine sichere Einhaltung eines rechten Winkels zwischen Zentralstrahl und Filmebene realisiert werden kann.

Bei der Rechtwinkel-/Paralleltechnik erfolgt zunächst eine Justierung des Filmes parallel zur Zahnachse und anschließend eine Ausrichtung des Tubus entlang einer Führungsstange. Hierdurch tritt der Zentralstrahl nahezu exakt im Winkel von 90° auf den Zahnfilm auf.

Der Erfindung liegt die Aufgabe zugrunde, eine Halterung für einen Sensor zu schaffen, wobei die Halterung eine Ablichtung eines oder mehrerer Zähne auf den Sensor in einer im wesentlichen unveränderten Größe ermöglicht und wobei eine möglichst genaue Justierung des Röntgentubus zu dem Sensor sowie eine genaue Winkelstellung des Röntgentubus zu dem Sensor erzielt wird. Insbesondere soll die Halterung sich für Statusaufnahmen und Kontrollaufnahmen von mehreren Seitenzähnen eignen.

Erfindungsgemäß wird diese Aufgabe dadurch gelöst, daß die Halterung wenigstens zwei im wesentlichen senkrecht zu der Bißplatte angeordnete Vorsprünge aufweist, wobei die Vorsprünge eine Länge aufweisen, die kleiner ist als es einer entsprechenden Höhenausdehnung des Sensors entspricht.

Hierdurch ist es möglich, ein Kabel seitlich heraus zu führen. Vorzugsweise werden die Vorsprünge so gewählt, daß sie eine Länge aufweisen, die geringer ist als ein Abstand zwischen einem Kabel des Sensors und einer Randfläche dieses Sensors.

Mit Hilfe der erfindungsgemäßen Halterung ist es möglich, Röntgenstrahlen außerhalb des Sensors wirksam abzuschirmen.

Die Vorsprünge werden insbesondere so dimensioniert, daß ihre Länge geringer ist als der Abstand zwischen dem Kabel und der seitlichen Randfläche bei handelsüblichen Sensoren.

Besonders zweckmäßig ist, daß die Vorsprünge so angeordnet sind, daß sie einen Abstand zueinander aufweisen, der im wesentlichen einer Längenausdehnung des Sensors entspricht.

Eine besonders wirksame und zuverlässige Halterung des Sensors läßt sich dadurch erzielen, daß der Sensor über einen Formschluß durch die Vorsprünge gehalten wird.

Ferner ist es vorteilhaft, daß die Halterung einen Flächenbereich aufweist, der sich im wesentlichen parallel zu der Bißplatte erstreckt und wobei die Vorsprünge in den Flächenbereich münden.

Eine unbeschädigte Zuführung mindestens eines Kabels zu dem Sensor läßt sich dadurch erzielen, daß die Bißplatte eine Dicke aufweist, die wenigstens einer Dicke des Kabels entspricht.

Insbesondere ist es vorteilhaft, daß die Bißplatte eine Dicke von wenigstens 4 mm aufweist.

Eine besonders zweckmäßige Ausführungsform der Halterung zeichnet sich dadurch aus, daß der Flächenbereich eine Breite von wenigstens 5 mm aufweist.

Es ist zweckmäßig, daß wenigstens einer der Vorsprünge eine Länge aufweist, die kürzer ist als es einem Abstand zwischen einem Kabelaustrittspunkt und einer Randfläche des Sensors entspricht. Die Vorsprünge weisen eine bevorzugte Länge von etwa 5 mm bis etwa 15 mm auf.

Besonders vorteilhaft ist, daß die Halterung über eine Führungsstange mit einem Visierring verbindbar ist, wobei die Verbindbarkeit insbesondere durch eine Führungsstange erzielt werden kann.

Durch den Visierring kann eine im Inneren des Röntgentubus befindliche Blende zur Eingrenzung des Nutzstrahlbündels entsprechend der Sensorfläche eingestellt werden.

Ein wirksamer Schutz von Patienten vor Röntgenstrahlung läßt sich dadurch erzielen, daß die Halterung mit einem Befestigungsmittel für eine Befestigung einer Abschirmblende verbindbar ist. Vorzugsweise wird das Befestigungsmittel durch die Führungsstange gebildet.

Ferner ist es vorteilhaft, daß die Abschirmblende und der Visierring durch ein einheitliches Bauteil gebildet werden.

Vorzugsweise sind das Befestigungsmittel und die Abschirmblende so gestaltet, daß eine Öffnung der Abschirmblende im wesentlichen parallel zu dem Sensor ausgerichtet ist.

Die Erfindung betrifft ferner ein Verfahren zum Verbinden eines mit wenigstens einem Kabel verbindbaren Sensors mit einer intraoralen Halterung. Dieses Verfahren zeichnet sich dadurch aus, daß der Sensor so mit der Halterung verbunden wird, daß das Kabel im Bereich einer seitlichen Randfläche und/oder im wesentlichen parallel zu einer oberen Randfläche aus dem Sensor austritt.

Die Halterung ist sowohl bei Aufnahmetechniken einsetzbar, bei denen eine genaue Größe des oder der Zähne unmittelbar durch flächengetreue Projektion erzielt wird, als auch bei Aufnahmetechniken, bei denen eine gleichbleibende Größe erst durch eine nachfolgende Bildverarbeitung erzielt wird.

Weitere Vorteile, Besonderheiten und zweckmäßige Weiterbildungen der Erfindung ergeben sich aus der nachfolgenden Darstellung eines bevorzugten Ausführungsbeispiels anhand der Zeichnungen.

Von den Zeichnungen zeigt:
- Fig. 1: eine Vorderansicht einer Halterung 10 in einer sich parallel zu einem Sensor 40 erstreckenden Ebene,
- Fig. 2: eine Seitenansicht der in Fig. 1 dargestellten Halterung 10 und
- Fig. 3: eine Draufsicht auf die in den Fig. 1 und 2 dargestellte Halterung 10.

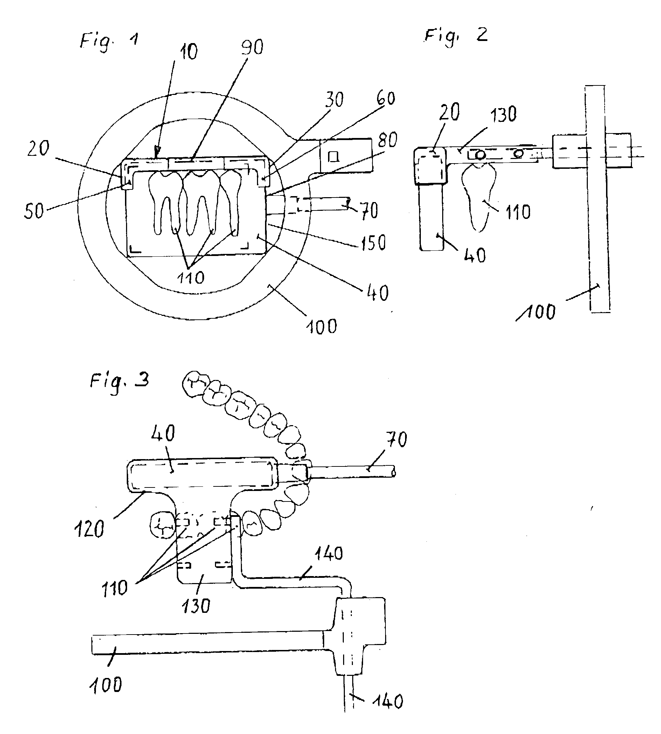

Die anhand der Fig. 1, 2 und 3 dargestellte Halterung 10 dient insbesondere für Statusaufnahmen von Zähnen. Die Halterung 10 weist Vorsprünge 20, 30 auf, die in einem Abstand voneinander angeordnet sind, der im wesentlichen so groß ist wie eine Längenausdehnung eines Sensors 40. Die Vorsprünge 20, 30 weisen vorzugsweise eine Breite auf, die an eine entsprechende Breite des Sensors 40 angepaßt ist. Es ist jedoch nicht erforderlich, daß die Vorsprünge breiter oder schmäler als der Sensor 40 sind. Die Vorsprünge 20, 30 halten den Sensor 40 durch einen Formschluß. Die Vorsprünge 20, 30 sind mit seitlichen Flanken 50, 60 versehen, so daß der Formschluß verstärkt wird. In diesem Falle sind die Vorsprünge 20, 30 wenigstens genauso dick wie der Sensor 40.

Es ist zweckmäßig, daß wenigstens der einem Kabel 70 zugewandte Vorsprung 30 eine Länge aufweist, die kürzer ist als es einem Abstand zwischen einem Kabelaustrittspunkt 80 und einer Randfläche 90 des Sensors 40 entspricht. Die Vorsprünge 20, 30 weisen eine bevorzugte Länge von etwa 5 mm bis etwa 15 mm auf.

Die Halterung 10 weist einen Elächenbereich 120 auf, der sich im wesentlichen parallel zu einer Bißplatte 130 erstreckt. Die Vorsprünge 20, 30 münden in den Flächenbereich.

Eine unbeschädigte Zuführung des Kabels 70 zu dem Sensor 40 läßt sich dadurch erzielen, daß die Bißplatte 130 eine Dicke aufweist, die wenigstens einer Dicke des Kabels 70 entspricht.

Insbesondere ist es vorteilhaft, daß die Bißplatte 130 eine Dicke von wenigstens 4 mm aufweist.

Die Halterung 10 ist insbesondere über eine Führungsstange mit einem Visierring 100 verbindbar. Der Visierring 100 ermöglicht eine genaue Justierung eines Röntgentubus auf die Mitte des Sensors 40 und gleichzeitig auf zu untersuchende Zähne 110.

Eine Verbindung der Halterung 10 mit dem Visierring 100 erfolgt vorzugsweise mittels eines Führungsmittels 140. Die Halterung 10 und das Führungsmittel 140 sind lösbar miteinander verbunden. Eine besonders einfache Verbindung zwischen der Halterung 10 und dem Visierring 100 läßt sich dadurch erzielen, daß die Halterung 10 wenigstens zwei Ausnehmungen aufweist. In die Ausnehmungen können komplementär geformte Vorsprünge des Führungsmittels 140 eingebracht werden. Bei dem Führungsmittel 140 handelt es sich im einfachsten Fall um eine handelsübliche Führungsstange.

### Bezugszeichenliste

- 10: Halterung
- 20: Vorsprung
- 30: Vorsprung
- 40: Sensor
- 50: seitliche Flanke
- 60: seitliche Flanke
- 70: Kabel
- 90: obere Randfläche des Sensors
- 80: Kabelaustrittspunkt
- 100: Visierring
- 110: Zahn
- 120: Flächenbereich
- 130: Bißplatte
- 140: Führungsmittel
- 150: seitliche Randfläche des Sensors

## Patentansprüche

1. Intraorale Halterung (10) mit einer Bißplatte, wobei die Halterung (10) zu einer Lagerung eines Sensors (40) dient, **dadurch gekennzeichnet,** das die Halterung (10) wenigstens zwei im wesentlichen senkrecht zu der Bißplatte (130) angeordnete Vorsprünge (20, 30) aufweist.

2. Halterung (10) nach Anspruch 1, **dadurch gekennzeichnet,** daß die Vorsprünge (20, 30) so angeordnet sind, daß sie einen Abstand zueinander aufweisen, der im wesentlichen einer Längenausdehnung des Sensors (40) entspricht.

3. Halterung (10) nach einem der Ansprüche 1 oder 2, **dadurch gekennzeichnet,** daß der Sensor (40) über einen Formschluß durch die Vorsprünge (20, 30) gehalten wird.

4. Halterung (10) nach einem der Ansprüche 1 bis 3, **dadurch gekennzeichnet,** daß sie einen Flächenbereich (120) aufweist, der sich im wesentlichen parallel zu der Bißplatte (130) erstreckt und wobei die Vorsprünge (20, 30) in den Flächenbereich (120) münden.

5. Halterung (10) nach Anspruch 4, **dadurch gekennzeichnet,** daß der Flächenbereich (120) eine Breite aufweist, die wenigstens einer Breite des Sensors (40) entspricht.

6. Halterung (10) nach einem der Ansprüche 1 bis 5, **dadurch gekennzeichnet,** daß die Vorsprünge (20, 30) eine Länge von 0,5 mm bis 5 mm aufweisen.

7. Halterung (10) nach einem der Ansprüche 1 bis 6, **dadurch gekennzeichnet,** daß sie über eine Führungsstange mit einem Visierring (100) verbindbar ist.

8. Halterung (10) nach einem der Ansprüche 1 bis 7, **dadurch gekennzeichnet,** daß sie mit einem Befestigungsmittel für eine Befestigung einer Abschirmblende verbindbar ist.

9. Halterung (10) nach Anspruch 8, **dadurch gekennzeichnet,** daß das Befestigungsmittel so gestaltet ist, daß eine Öffnung der Abschirmblende im wesentlichen parallel zu dem Sensor (40) ausgerichtet ist.

10. Halterung (10) nach einem der Ansprüche 1 bis 9, **dadurch gekennzeichnet,** daß die Bißplatte (130) eine Dicke aufweist, die wenigstens einer Dicke eines Kabels (70) entspricht.

11. Halterung (10) nach Anspruch 10**,dadurch gekennzeichnet,** daß die Bißplatte (130) eine Dicke von wenigstens 4 mm aufweist.

12. Halterung (10) nach einem der Ansprüche 1 bis 11, **dadurch gekennzeichnet,** daß sie genau zwei Vorsprünge (20, 30) aufweist.

13. Halterung (10) nach einem der Ansprüche 1 bis 12, **dadurch gekennzeichnet,** daß wenigstens ein einem Kabel (70) zugewandter Vorsprung (30) eine Länge aufweist, die kürzer ist als es einem Abstand zwischen einem Kabelaustrittspunkt (80) und einer Randfläche (90) des Sensors 40 entspricht.

14. Verfahren zum Verbinden eines mit wenigstens einem Kabel (70) verbindbaren Sensors (40) mit einer intraoralen Halterung (10), **dadurch gekennzeichnet,** daß der Sensor (40) so mit der Halterung (10) verbunden wird, daß das Kabel (70) im Bereich einer seitlichen Randfläche (150) und/oder im wesentlichen parallel zu einer oberen Randfläche (90) aus dem Sensor (40) austritt.
